# EUROPEAN PATENT APPLICATION

(11) **EP 2 034 307 A1**
(43) Date of publication of application: **11.03.2009**
(21) Application number: 08163633.4
(22) Date of filing: 03.09.2008
(51) Int. Cl.: G01N 33/00, G01L 19/00, G01L 19/06, H01M 8/04

(54) **Gas physical quantity detecting device, fuel cell system and vehicle**

(30) Priority: 05.09.2007 JP 2007230161
(71) Applicant: Nissan Motor Co., Ltd., Kanagawa 221-0023 (JP)
(72) Inventor: Hatta, Kentaro c/o Nissan Motor Co., Ltd., Atsugi-shi, Kanagawa 243-0123 (JP); Hirose, Katsutoshi c/o Nissan Motor Co., Ltd., Atsugi-shi, Kanagawa 243-0123 (JP)
(74) Representative: Holmes, Matthew William

(57) **Abstract**

A gas physical quantity detecting device (1) is basically provided with detection element (5), a detection element chamber (7) and a filter (6). The detection element (5) is configured to detect a physical quantity of a gas flowing through a gas flow passage (2). The detection element chamber (7) contains the detection element (5) and is configured to supply gas from inside the gas flow passage (2) to the detection element (5). The filter (6) is arranged between the gas flow passage (2) and the detection element chamber (7). The filter (6) has a predetermined thickness with the filter (6) being made of a non-hydrophobic material.

## Description

The present invention generally relates to a gas physical quantity detecting device for detecting a physical quantity of a gas. Aspects of the invention relate to a device, to an apparatus, to a system and to a vehicle.

In conventional technology, a pressure sensor device can be provided to detect gas pressure in a pipe. In one type of pressure sensor device, a filter with an oil impregnated metal mesh or non-woven fabric is provided in an inlet of a sensor element chamber to prevent liquid water or contaminants from entering into the sensor element chamber. One example of this type of pressure sensor device is disclosed in Japanese Laid-Open Patent Publication No. 11-30535 (see, paragraphs 0054 to 0056).

It has been discovered that with a conventional pressure sensor device, as discussed above, water vapor enters into the sensor element chamber through the filter when the device is in a high humidity and high temperature environment. If the ambient temperature decreases, then water condensation (dew) will occur inside the sensor element chamber. Dew or water that has condensed inside the sensor element chamber tends to adhere to the sensor element or form a liquid film on the filter surface due to surface tension. When this occurs, the gas targeted for pressure detection can not reach the sensor element in an effective manner and the gas pressure can not be detected accurately.

It is an aim of the present invention to address this issue and to improve upon known technology. Embodiments of the invention may provide a gas physical quantity detecting device that can detect a physical quantity of a gas accurately without the occurrence of poor detection results caused by water condensation, or a fuel cell system and a vehicle in which the gas physical quantity detecting device is employed. Other aims and advantages of the invention will become apparent from the following description, claims and drawings.

Aspects of the invention therefore provide an apparatus, a system and a vehicle as claimed in the appended claims.

According to another aspect of the invention for which protection is sought, there is provided a gas physical quantity detecting device comprising a detection element configured to detect a physical quantity of a gas flowing through a gas flow passage, a detection element chamber containing the detection element and configured to supply gas from inside the gas flow passage to the detection element, and a filter arranged between the gas flow passage and the detection element chamber, the filter having a predetermined thickness with the filter being made of a non-hydrophobic material.

In an embodiment, the non-hydrophobic material has fibers interwoven in a non-uniform manner such that the filter has a three-dimensional form.

In an embodiment, the fibers of the filter are made of stainless steel having a composition of one of SUS316 and SUS304.

In an embodiment, the fibers are arranged to form gaps between the fibers on a surface of the filter that faces the detection element chamber in which the gaps are smaller than a maximum flame extinguishing diameter of a hydrogen flame.

In an embodiment, the filter has a multiple layered structure with a space provided between adjacent layers.

In an embodiment, the filter has a fiber density at a peripheral portion of the filter that is lower than a fiber density at a middle portion of the filter.

In an embodiment, the filter has a fiber density on a side of the filter nearer to the detection element chamber that is lower than a fiber density on a side of the filter nearer to the gas passage.

In an embodiment, the filter has a protruding part that protrudes into the gas flow passage.

In an embodiment, the filter has a recessed shaped surface that faces the detection element chamber.

In an embodiment, the filter at least partially extends along an internal side wall surface of the detection element chamber.

In an embodiment, the detection element chamber has an internal wall surface with a water repellent treatment.

In an embodiment, the detection element chamber has an internal wall surface with a hydrophilic treatment.

In an embodiment, the detection element includes a cover member. The detection element chamber may have an internal wall surface with a surface roughness that is larger than a surface roughness of a surface of the cover member that faces the detection element chamber.

In an embodiment, the detection element includes a cover member with a surface of the cover member facing the detection element chamber having a water repellent treatment.

In an embodiment, the detection element includes a first sensor in a first cover and a second sensor in a second cover, with the first cover having a gas introducing hole and the second sensor being a reference sensor with the second cover containing a reference gas.

In an embodiment, the first and second cover members are spaced apart with a gap therebetween.

In an embodiment, the first and second cover members are spaced inwardly from an internal side wall of the detection element chamber.

According to a further aspect of the invention for which protection is sought, there is provided a fuel cell system provided with the gas physical quantity detecting device as set out in any of the previous paragraphs, the fuel cell system comprising a fuel cell with a fuel electrode, a pipe arranged to supply a gas to the fuel electrode of the fuel cell and a pipe arranged to discharge the gas from the fuel electrode, the gas physical quantity detecting device being connected to at least one of the pipes.

According to a still further aspect of the invention for which protection is sought, there is provided a fuel cell system provided with the gas physical quantity detecting device as set out in any of the previous paragraphs, the fuel cell system comprising a fuel cell that receives hydrogen, with the gas physical quantity detecting device being arranged in an area to detect leakage of hydrogen from the fuel cell system.

According to yet another aspect of the invention for which protection is sought, there is provided a vehicle provided with the fuel cell system as recited in either of the previous two paragraphs, the vehicle comprising a vehicle body supporting the fuel cell system and at least one drive wheel arranged to use electric power generated by the fuel cell system as a drive source.

For example, a gas physical quantity detecting device may comprise a detection element, a detection element chamber and a filter. The detection element may be configured to detect a physical quantity of a gas flowing through a gas flow passage. The detection element chamber may contain the detection element and configured to supply gas from inside the gas flow passage to the detection element. The filter may be arranged between the gas flow passage and the detection element chamber. The filter may have a predetermined thickness with the filter being made of a non-hydrophobic material.

Within the scope of this application it is envisaged that the various aspects, embodiments, examples, features and alternatives set out in the preceding paragraphs, in the claims and/or in the following description and drawings, may be taken individually or in any combination thereof.

The present invention will now be described, by way of example only, with reference to the accompanying drawings, in which:
Figure 1 is a partial cross sectional view of a gas physical quantity detecting device in accordance with a first embodiment;
Figure 2 is a partial cross sectional view showing a variation of the filter of the gas physical quantity detecting device shown in Figure 1;
Figure 3 is a partial cross sectional view showing a variation of the filter of the gas physical quantity detecting device shown in Figure 1;
Figure 4 is a partial cross sectional view showing a variation of the filter of the gas physical quantity detecting device shown in Figure 1;
Figure 5 is a partial cross sectional view showing a variation of the filter of the gas physical quantity detecting device shown in Figure 1;
Figure 6 is a partial cross sectional view of a gas physical quantity detecting device in accordance with a second embodiment;
Figure 7 is a schematic diagram of a vehicle with a fuel cell system in which the gas physical quantity detecting device has been employed;
Figure 8 is a schematic diagram of a vehicle with a fuel cell system in which the gas physical quantity detecting device has been employed; and
Figure 9 is a schematic diagram of a vehicle with a fuel cell system in which the gas physical quantity detecting device has been employed.

It will be apparent to those skilled in the art from this disclosure that the following descriptions of the embodiments of the present invention are provided for illustration only and not for the purpose of limiting the invention as defined by the appended claims and their equivalents.

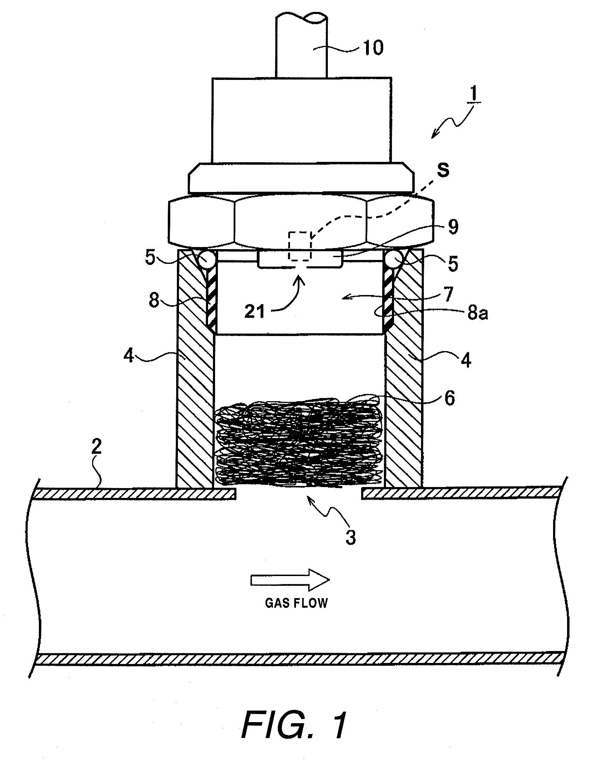

Referring initially to Figure 1, a gas physical quantity detecting device is illustrated in accordance with a first embodiment. The constituent features of according to the first embodiment of the present invention will now be explained with reference to Figure 1. As explained below, in the illustrated embodiment, a physical quantity of a gas can be detected accurately without the occurrence of poor detection results caused by condensed water. The gas physical quantity detecting device 1 detects a concentration of gas flowing inside a gas pipe 2.

The gas physical quantity detecting device 1 is mounted around an opening 3 in the gas pipe 2 by a sensor mounting part 4 of the gas physical quantity detecting device 1. The sensor mounting part 4 is a tubular member that is formed around the opening 3 in the gas pipe 2. In this embodiment, an O-ring 5 is arranged between the gas physical quantity detecting device 1 and the sensor mounting part 4 to provide an airtight seal and prevent gas from leaking between the gas physical quantity detecting device 1 and the sensor mounting part 4.

The gas physical quantity detecting device 1 further includes a filter 6 disposed in a bottom portion of the sensor mounting part 4, a detection element chamber 7 defined by an interior of a tubular sensor enclosure 8, an element cover 9 and an electrical wiring harness 10. The element cover 9 is arranged in an upper portion of the inside of the detection element chamber 7 and contains inside a resistance temperature sensor S. The electrical wiring harness 10 receives and transmits a detection signal from the resistance temperature sensor, and supplies electric power to the resistance temperature sensor S. The resistance temperature sensor S detects a concentration of gas that passes from the gas pipe 2 to the resistance temperature sensor S through the opening 3, the filter 6, and the detection element chamber 7. The concentration is detected based on a temperature change of the resistance temperature sensor S. Although in this embodiment a resistance temperature sensor is provided inside the element cover 9 to detect a concentration of the gas, it is also acceptable to change the sensor configuration inside the element cover 9 so as to detect a temperature, pressure or other physical quantity. In this embodiment, the element cover 9 is provided with a small opening 21 for introducing the gas to the resistance temperature sensor S.

The filter 6 is made by interweaving non-hydrophobic fibers in a non-uniform manner such that the filter 6 of non-hydrophobic fibers has a three-dimensional form. In other words, the filter 6 has continuous passageways for gas to pass through the filter 6 from the gas pipe 2 to the detection element chamber 7. However, theses continuous passageways of the filter 6 are non-linear passageways. If the gas of which a physical quantity is being detected contains hydrogen, then it is advantageous for the fiber material to be one of the following stainless steels: SUS316 (a stainless steel 18% Cr, 12% Ni, 2% Mo, 0.08% or less of C added thereto), SUS316L (a stainless steel having 18% Cr, 12% Ni, 2% Mo, 0.03% or less of C added thereto), or SUS304 (a stainless steel containing 18% Cr and 8% Ni). By using one of these fiber materials, the filter 6 can be prevented from degrading due to exposure to hydrogen and the filter 6 can be used continuously while exposed to hydrogen without being replaced.

With the gas physical quantity detecting device 1, as described above, if liquid water is contained in the gas flowing through the gas pipe 2, the liquid water will be blocked by the filter 6 and prevented from entering into the detection element chamber 7. As a result, liquid water can be prevented from adhering to the element cover 9 and causing a poor detection result to occur. Additionally, liquid water is prevented from clogging the filter 6 and inhibiting the exchange of gas between the gas pipe 2 and the detection element chamber 7. As a result, the physical quantity of the gas can be detected accurately.

Meanwhile, if water vapor is contained in the gas flowing through the gas pipe 2, the water vapor will pass through the filter 6 and enter the detection element chamber 7. Consequently, there is the possibility that water vapor inside the detection element chamber 7 will condense and cause condensed water (dew) to exist inside the detection element chamber 7. However, since the filter 6 is made by interweaving a non-hydrophobic fiber(s) in a non-uniform manner such that the non-hydrophobic fiber(s) has a three-dimensional form, the surface of the filter 6 is not a flat plane but, instead, has fine indentations and protrusions. The internal structure of the filter 6 is such that there are continuous air voids whose sizes change as they interconnect. These air voids form a plurality of paths through which gas can flow. Thus, an air layer occupies a larger portion of the surface of the filter 6 than in the conventional technology.

In general, the surface tension of water is determined by a contact angle of the water with a surface. In the case of the filter 6, the non-hydrophobic fiber(s) forms many contact surfaces with water droplets in the filter 6. In other words, the non-linear passageways of the filter 6 forms many contact surfaces with water droplets in the filter 6. When water is spread over a plurality of objects (i.e., fibers), the overall contact angle is calculated as a sum of the individual contact angles weighted based on surface area. Since the contact angle of water with respect to air is 0, the contact angle of water with respect to the filter 6 that is occupied by an air layer to a large degree is smaller than the contact angle with respect to a conventional filter. Consequently, a liquid film does not form on the surface of the filter due to surface tension and condensed water infiltrates to the interior of the filter 6 by capillary action. Although a similar effect can be obtained by applying a hydrophilic treatment to the filter, it is necessary to raise the temperature or apply some other process in order to activate the hydrophilic treatment, which causes the cost to increase.

Since the internal structure of the filter 6 is such that there are continuous air voids whose sizes change as they interconnect, any condensed water that is absorbed into the filter 6 gathers in places where the surface tension is strong and leaves places that are not filled with liquid water. Additionally, since gas is constantly entering and exiting the upper and lower surfaces of the filter 6, a situation in which all of the gas paths inside the filter 6 become clogged does not occur. Even if water condenses inside the detection element chamber 7, a situation in which gas is blocked to the inside of the detection element chamber 7 does not occur and a physical quantity of the gas can be detected accurately.

It is advantageous for the sizes of the air voids existing on a surface of the filter 6 facing toward the detection element chamber 7 to be smaller than a maximum flame extinguishing diameter (approximately 0.8 mm at atmospheric pressure) of a hydrogen flame at a pressure of a gas flowing inside the gas pipe 2. Thus constructed, even if a spark occurs inside the detection element chamber 7 while the device is being used in a hydrogen atmosphere, the flame will not spread to the gas pipe 2 side and the flame can be suppressed inside the detection element chamber 7.

In order that condensed water occurring inside the detection element chamber 7 will not form a liquid film on an internal wall surface 8a of the sensor enclosure 8, a hydrophilic treatment is applied to the internal wall surface 8a or the roughness of the internal wall surface 8a is made to be larger than the roughness of the surface of the element cover 9. When such a constituent feature is adopted, even if water condensation occurs inside the detection element chamber 7, liquid droplets will not adhere to the internal wall surface 8a. Additionally, since the roughened internal wall surface 8a of the detection element chamber 7 serves as a water condensation kernel, condensed water does not develop on the surface of the element cover 9. Therefore, poor detection results caused by liquid water adhered to the element cover 9 can be prevented. A similar technical effect can be obtained by applying a water repellant treatment to the internal wall surface 8a because doing so will prevent liquid droplets from adhering to the internal surface 8a.

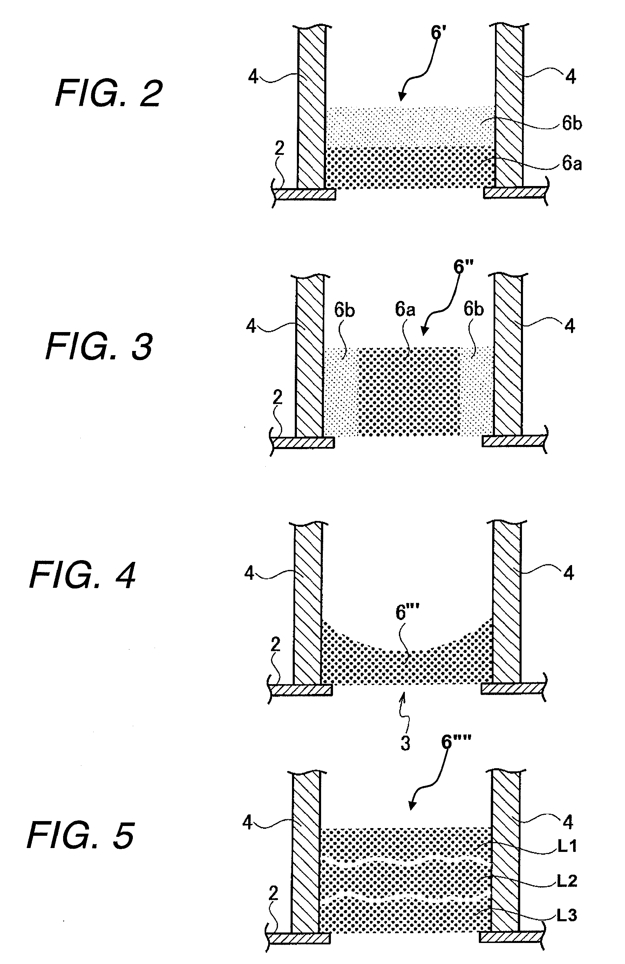

Variation of the filter 6 of Figure 1 will now be explained with reference to Figure 2 to 5. Since these variations use the same configurations shown in Figure 1, except for the filter construction, only parts of the gas pipe and the sensor mounting part will be illustrated. The remaining parts of the gas physical quantity detecting device 1 are the same as in Figure 1. Also in each of these variations, the filters 6' to 6"" are made of the same material as the filter 6 of the first embodiment, as discussed above, except that their densities and/or shapes are changed.

In the filter shown in Figure 2, the fiber density varies along an axial direction of the filter 6'. More specifically, the filter 6' has a first filter section 6a with a first fiber density and a second filter section 6b with a second fiber density that is different than the first fiber density of the first filter section 6a. The second filter section 6b is arranged nearer to the detection element chamber 7 than the first filter section 6a, which is arranged nearer to the gas pipe 2. The second fiber density of the second filter section 6b is smaller than the first fiber density of the first filter section 6a. With this filter configuration, since the second fiber density of the second filter section 6b of the detection element chamber 7 is small, condensed water that develops inside the detection element chamber 7 can be quickly absorbed into the second filter section 6b such that condensed water can be discharged effectively.

In the filter 6" shown in Figure 3, the fiber density varies in radial direction of the filter 6". More specifically, the filter 6" has the first filter section 6a with the first fiber density located in the center with the second filter section 6b with the second fiber density surrounding the first filter section 6a. The second fiber density of the second filter section 6b, which is disposed in a peripheral section, is smaller than the first fiber density of the first filter section 6a, which is disposed in a middle section. When gas is exchanged between the gas pipe 2 and the detection element chamber 7, the flow speed of the gas is zero (0) at the internal wall surface of the sensor mounting part 4. Thus, with this filter configuration, the discharge of water from the peripheral second filter section 6b can be promoted because the fiber density of the peripheral second filter section 6b is smaller than the fiber density of the middle first filter section 6a.

In the filter 6"' shown in Figure 4, the surface of the filter 6"' has a concave shape on the side facing the detection element chamber 7. With this filter configuration, the surface area of the filter 6"' on the side facing the detection element chamber 7 is larger than if the surface was planar. Consequently, condensed water produced inside the detection element chamber 7 can be discharged even more effectively.

In the filter 6"" shown in Figure 5, the filter 6"" has a multiple layered structure having gaps in-between layers (the example shown in Figure 5 has a three layered structure comprising three filters L1, L2 and L3). With this filter configuration, the volume of the air voids in the filter 6"" can be increased. As a result, even if a large amount of condensed water develops or a large amount of liquid water flows in from the gas pipe 2, the penetration of liquid water into the detection element chamber 7 can be prevented in an effective manner.

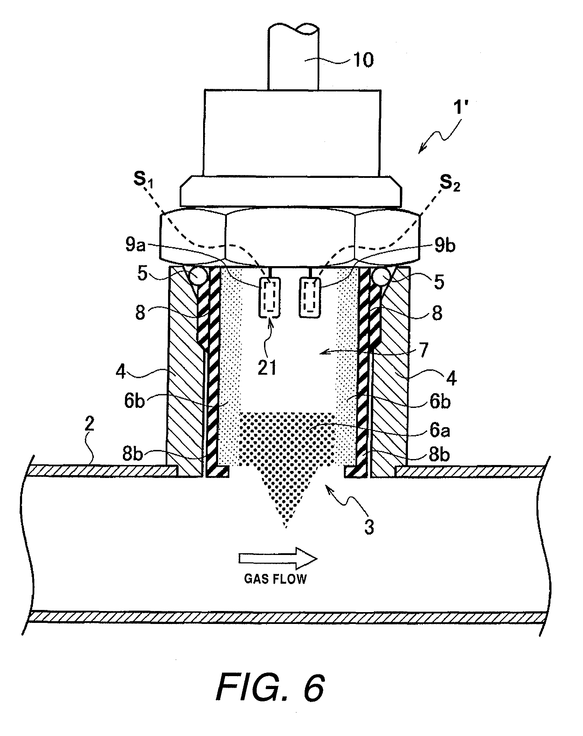

Referring now to Figure 6, a gas physical quantity detecting device 1' in accordance with a second embodiment will now be explained. In view of the similarity between the first and second embodiments, the parts of the second embodiment that are identical to the parts of the first embodiment will be given the same reference numerals as the parts of the first embodiment. Moreover, the descriptions of the parts of the second embodiment that are identical to the parts of the first embodiment may be omitted for the sake of brevity.

As shown in Figure 6, there are mainly three basic differences between the first and second embodiments. First, in the second embodiment, a two part filter of the gas physical quantity detecting device 1' includes a first filter section 6a with the first fiber density located in the center with a second filter section 6b with a second fiber density surrounding the first filter section 6a. Second, in the second embodiment, the sensor enclosure 8 of the gas physical quantity detecting device 1' further includes an inner tubular member 8b. Third, in the second embodiment, the element cover of the gas physical quantity detecting device 1' has a detection element cover 9a and a reference element cover 9b. The parts that are different will now be explained.

In this second embodiment, the inner tubular member 8b is provided inside on the internal wall of the sensor enclosure 8. The inner tubular member 8b extends to a bottom portion of the sensor mounting part 4. Thus, the inner tubular member 8b forms the detection element chamber 7. The first filter section 6a is arranged in a bottom portion of the inner tubular member 8b. The second filter section 6b is arranged on an internal wall surface of the inner tubular member 8b and extends along the entire area of the internal wall surface of the inner tubular member 8b. Although in this embodiment the first filter section 6a is arranged at least partially inside the detection element chamber 7, it is acceptable for the filter to be arranged outside the detection element chamber 7 (as shown in Figure 1) so long as the filter is between the gas pipe 2 and the detection element chamber 7.

The first filter section 6a is made of the same material as the filter 6 of the first embodiment as discussed above. The first filter section 6a is configured and arranged such that a surface thereof that is nearer to the gas pipe 2 protrudes into the gas pipe 2 through the opening 3. By configuring the first filter section 6a such that the surface thereof facing the gas pipe 2 protrudes into the gas pipe 2, moisture absorbed by the first filter section 6a can be carried away by gas flowing through the gas pipe 2. As a result, liquid water inside the detection element chamber 7 can be discharged in an effective manner.

The second filter section 6b is made of the same material as the filter 6 of the first embodiment as discussed above, and is provided on the internal wall surface of the inner tubular member 8b. Providing the second filter section 6b enables condensed water inside the detection element chamber 7 to be absorbed at the side wall portions in addition to the bottom portion of the detection element chamber 7. As a result, liquid water can be prevented from adhering to a detection element cover 9a and a reference element cover 9b, which are described later. Since the first filter section 6a serves as a kernel for water condensation, condensation of water on the surfaces of the element cover 9a and the reference element cover 9b can be prevented. The second fiber density of the second filter section 6b, which is disposed in a peripheral section, is smaller than the first fiber density of the first filter section 6a, which is disposed in a middle section, similar to Figure 3.

The detection element cover 9a and the reference element cover 9b are arranged inside the detection element chamber 7. The detection element cover 9a contains a resistance temperature sensor S₁ for gas detection inside. The detection element cover 9a has a narrow hole 21. The reference element cover 9b contains a reference resistance temperature sensor S₂ and a reference (comparative) gas. The reference element cover 9b is sealed. Together, the sensors S₁ and S₂ form a thermal conductivity hydrogen sensor that detects a hydrogen concentration based on a temperature change of the gas detection resistance temperature sensor S₁ (which changes in accordance with the hydrogen concentration of the gas) while compensating based on a temperature change of the reference resistance temperature sensor S₂. With this configuration, the hydrogen concentration can be detected accurately even if the hydrogen concentration is high, there is no oxygen present, and water vapor exists.

When the thermal conductivity hydrogen sensor (S₁ and S₂) is used, if there is a flow of gas inside the detection element cover 9a, then the detection value will be affected because the temperature of the gas detection resistance temperature sensor S₁ will change due to the heat transfer coefficient in addition to the thermal conductivity. Therefore, it is advantageous to take such measures as making the thickness of the first filter section 6a ten (10) mm or larger and making the diameter of the gas pipe 2 larger at the position where the gas physical quantity detecting device 1' is mounted than at other positions in order to suppress the occurrence of gas flow inside the detection element cover 9a.

When the first filter section 6a has absorbed liquid water, there is the possibility that liquid water will be splashed from the first filter section 6a toward the detection element chamber 7 if the flow rate of gas inside the gas pipe 2 increases abruptly. Therefore, it is beneficial for a gap of several millimeters to be provided between the first filter section 6a and the detection element cover 9a. By providing such a gap, splashed water can be prevented from adhering to the surface of the detection element cover 9a.

It is advantageous to apply a water repellent treatment to the surfaces of both the detection element cover 9a and the reference element cover 9b. By applying a water repellent treatment to both surfaces, liquid droplets can be prevented from adhering to the surfaces of the detection element cover 9a and the reference element cover 9b even when liquid water absorbed by the first filter section 6a is splashed onto the surfaces of the detection element cover 9a and the reference element cover 9b or when water condenses on the surfaces of the detection element cover 9a and the reference element cover 9b. As a result, poor detection results can be prevented.

It is beneficial for a gap to be provided between the detection element cover 9a and the reference element cover 9b. By providing a gap, even if liquid droplets adhere to the surface of either the detection element cover 9a or the reference element cover 9b, a situation in which poor detection results occur due to the formation of a liquid film spanning between the detection element cover 9a and the reference element cover 9b can be prevented.

It is beneficial to provide a gap between the internal wall of the detection element chamber 7 and both the detection element cover 9a and the reference element cover 9b. By providing such a gap, even if liquid droplets adhere to the internal wall of the detection element chamber 7 and to the surface of either the detection element cover 9a or the reference element cover 9b, a situation in which poor detection results occur due to the formation of a liquid film spanning between the internal wall of the detection element chamber 7, the detection element cover 9a, and the reference element cover 9b can be prevented.

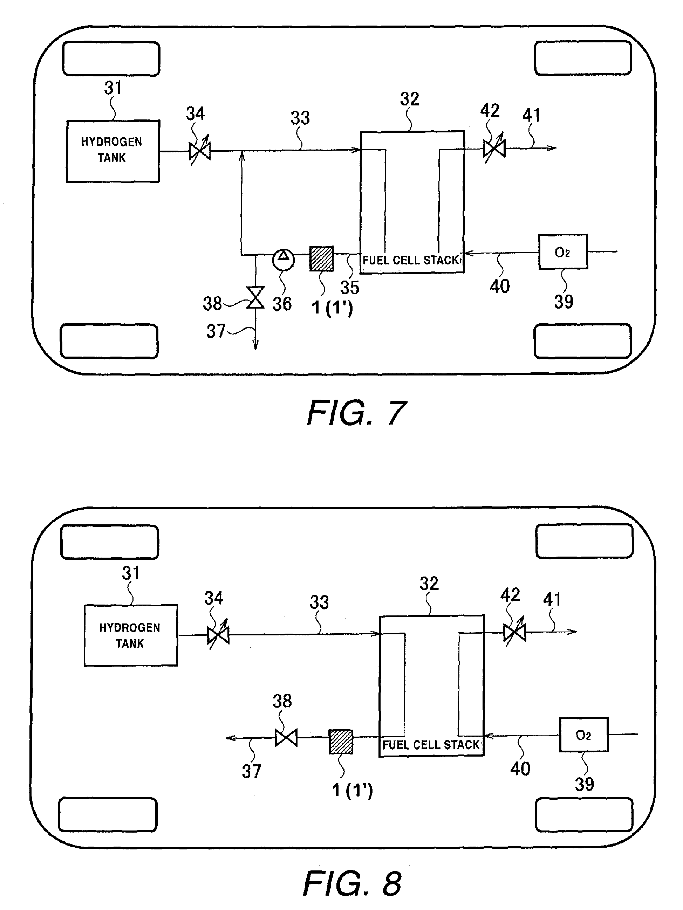

The gas physical quantity detecting devices 1 and 1' (including the filter variations of Figures 2 to 5) can be employed in a fuel cell system of a vehicle like those shown in Figures 7 to 9 to generate electric power for driving a vehicle. Thus, the fuel cell system of Figures 7 to 9 function a drive source for the vehicle.

Three examples 1 to 3 in which one of the gas physical quantity detecting devices 1 and 1' (including the filter variations of Figures 2 to 5) is employed in a fuel cell system will now be explained with reference to Figures 7 to 9. When one of these fuel cell systems is installed in a vehicle, it is advantageous for the gas physical quantity detecting devices 1 and 1' (including the filter variations of Figures 2 to 5) to extend in a generally vertically upward direction form the gas pipe 2. It is not necessary, however, for the gas pipe 2 to be arranged horizontally. It is acceptable for the gas pipe 2 to be slanted. It is also advantageous for the gas physical quantity detecting devices 1 and 1' (including the filter variations of Figures 2 to 5) to be arranged such that its tilt angle stays within ± 40° when the vehicle tilts.

### FIRST EXAMPLE

Referring now to Figure 7, the fuel cell system basically includes a hydrogen tank 31, a fuel cell stack 32, a hydrogen supply pipe 33, and a hydrogen pressure reducing valve 34. The fuel cell stack 32 includes a plurality of stacked fuel cells with each stacked fuel cell having a fuel electrode and an oxidant electrode to generate electricity using hydrogen supplied to the fuel electrode and air supplied to the oxidant electrode. Each of the fuel cells is a solid polymer fuel cell comprising a membrane-electrode composite body and separators. The membrane-electrode composite body includes catalyst layers of the fuel electrode and the oxidant electrode and a solid electrolyte membrane pinched between gas dispersion electrodes. The separators have gas flow passages for supplying hydrogen to the fuel electrode and air to the oxidant electrode. The separators are arranged to sandwich the membrane-electrode composite body. The electrochemical reactions that take place at the fuel electrode and the oxidant electrode and the electrochemical reaction of the fuel cell as a whole are shown below in the chemical equations (1) to (3).

Fuel electrode: H₂ → 2H⁺ + 2e- (1)

Oxidant electrode: ½O₂ + 2H⁺ + 2e⁻ → H₂O (2)

Overall: H₂ +½O₂ → H₂O (3)

The hydrogen pressure reducing valve 34 serves to reduce the pressure of the high pressure hydrogen inside the hydrogen tank 31 to a pressure that is well suited to the operating conditions of the fuel cell tank 32. The hydrogen supply pipe 33 serves to deliver the hydrogen to the fuel electrode of the fuel cell stack 32.

A hydrogen circulation pipe 35 and a circulation pump 36 are provided on an outlet side of the fuel electrode and serve to circulate gas discharged from the fuel electrode to the hydrogen supply pipe 33. A hydrogen discharge pipe 37 extends from the hydrogen circulation pipe 35 to discharge gas from the fuel electrode to the outside. A hydrogen discharge on-off valve 38 opens and closes the hydrogen discharge pipe 37.

The fuel cell system also has a compressor 39 and an air supply pipe 40. The compressor 39 serves to supply compressed air to the oxidant electrode of the fuel cell stack 32 through the air supply pipe 40. An air discharge pipe 41 extends from the air discharge pipe 41 to discharge air from the oxidant electrode to the outside. An air discharge on-off valve 42 opens and closes the air discharge pipe 41.

In this fuel cell system, one of the gas physical quantity detecting devices 1 and 1' (including the filter variations of Figures 2 to 5) is provided on the hydrogen discharge pipe 35 for detecting a concentration of hydrogen contained in a gas discharged from the fuel electrode. The fuel cell system is configured to control the opening degrees of the hydrogen pressure regulating valve 34 and the hydrogen discharge on-off valve 38 based on the hydrogen concentration detected by the gas physical quantity detecting device 1 or 1'.

Although one of the gas physical quantity detecting devices 1 and 1' (including the filter variations of Figures 2 to 5) is provided upstream of the circulation pump 36 in the first example, it is acceptable for one of the gas physical quantity detecting devices 1 and 1' (including the filter variations of Figures 2 to 5) to be provided downstream of the circulation pump 36 or downstream of the hydrogen pressure regulating valve 34. However, if one of the detecting devices is provided on the downstream side of the circulation pump 36, then the detection error will be larger than when the detection device is provided on the upstream side of the circulation pump 36 because the gas flow speed is higher on the downstream side.

Meanwhile, if the detection device is provided in the hydrogen supply pipe 33, then the detection error will be larger than when the detection device 1 is provided on the upstream side of the circulation pump 36 because a high hydrogen concentration will be detected at all times and the gas flow rate is higher in the hydrogen supply pipe 33. As a result, it is beneficial for the gas physical quantity detecting devices 1 and 1' (including the filter variations of Figures 2 to 5) to be provided on the upstream side of the circulation pump 36.

### SECOND EXAMPLE

Referring now to Figure 8, the fuel cell system does not have the hydrogen circulation pipe 35 or the circulation pump 36 of the first example. The hydrogen discharged from the fuel electrode is discharged directly to the outside of the system through the hydrogen discharge pipe 37 and the hydrogen discharge on-off valve 38. In this fuel cell system, similarly to first example, one of the gas physical quantity detecting devices 1 and 1' (including the filter variations of Figures 2 to 5) is provided on the hydrogen discharge pipe 35 for detecting a concentration of hydrogen contained in a gas discharged from the fuel electrode. The fuel cell system is configured to control the opening degrees of the hydrogen pressure regulating valve 34 and the hydrogen discharge on-off valve 38 based on the hydrogen concentration detected by the gas physical quantity detecting device.

### THIRD EXAMPLE

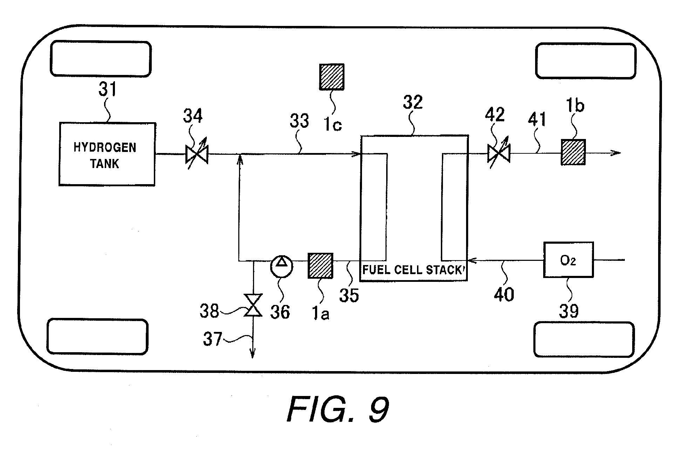

Referring now to Figure 9, the fuel cell system has generally the same constituent features as the fuel cell system shown in Figure 7. However, in this fuel cell system, three gas physical quantity detecting devices 1a, 1b, and 1c are provided. Each of the gas physical quantity detecting devices 1 a, 1 b, and 1 c can be any one of the gas physical quantity detecting devices 1 and 1' (including the filter variations of Figures 2 to 5) as discussed above. The gas physical quantity detecting device 1a is provided in the hydrogen discharge pipe 35. The gas physical quantity detecting device 1b is provided in the air discharge pipe 41. The gas physical quantity detecting device 1c is provided in a position outside of the fuel cell system. Thus, a concentration of hydrogen contained in gas discharged from the fuel electrode, a concentration of hydrogen contained in gas discharged from the oxidant electrode, and a concentration of hydrogen contained in gas discharged from the fuel cell system are detected. The position outside of the fuel cell system can be, for example, near the fuel cell stack 32 or inside a vehicle cabin if the fuel cell system is installed in a vehicle. With this configuration, a leakage of hydrogen into the air discharge pipe 41 (through which high-humidity gas flows) or a leakage of hydrogen from the fuel cell system can be detected.

While only selected embodiments have been chosen to illustrate the present invention, it will be apparent to those skilled in the art from this disclosure that various changes and modifications can be made herein without departing from the scope of the invention as defined in the appended claims. For example, the size, shape, location or orientation of the various components can be changed as needed and/or desired. Components that are shown directly connected or contacting each other can have intermediate structures disposed between them. The functions of one element can be performed by two, and vice versa. The structures and functions of one embodiment can be adopted in another embodiment. It is not necessary for all advantages to be present in a particular embodiment at the same time. Every feature which is unique from the prior art, alone or in combination with other features, also should be considered a separate description of further inventions by the applicant, including the structural and/or functional concepts embodied by such feature(s). Thus, the foregoing descriptions of the embodiments according to the present invention are provided for illustration only, and not for the purpose of limiting the invention as defined by the appended claims and their equivalents.

This application claims priority from Japanese Patent Application No. 2007-230161, filed 5th September 2007, the contents of which are expressly incorporated herein by reference.

## Claims

1. An apparatus, comprising:
detection means for detecting a physical quantity of a gas flowing through a gas flow passage;
chamber means for containing the detection means and arranged to supply gas from inside the gas flow passage to the detection means; and
filter means arranged between the gas flow passage and the chamber means, the filter means having a predetermined thickness and being made of a non-hydrophobic material.

2. An apparatus as claimed in claim 1, wherein the non-hydrophobic material has fibers interwoven in a non-uniform manner such that the filter means has a three-dimensional form.

3. An apparatus as claimed in claim 2, wherein the fibers of the filter means comprise a stainless steel having a composition of one of SUS316 and SUS304.

4. An apparatus as claimed in claim 2 or claim 3, wherein the fibers are arranged to form gaps between the fibers on a surface of the filter means that faces the chamber means in which the gaps are smaller than a maximum flame extinguishing diameter of a hydrogen flame.

5. An apparatus as claimed in any preceding claim, wherein the filter means has a multiple layered structure with a space provided between adjacent layers.

6. An apparatus as claimed in claim 2 or any claim dependent on claim 2, wherein the filter means:
has a fiber density at a peripheral portion of the filter means that is lower than a fiber density at a middle portion of the filter means; and/or
has a fiber density on a side of the filter means nearer to the chamber means that is lower than a fiber density on a side of the filter means nearer to the gas passage.

7. An apparatus as claimed in any preceding claim, wherein the filter means:
has a recessed shaped surface that faces the chamber means; and/or
at least partially extends along an internal side wall surface of the chamber means.

8. An apparatus as claimed in any preceding claim, wherein the chamber means has an internal wall surface with a water repellent treatment and/or a hydrophilic treatment.

9. An apparatus as claimed in any preceding claim, wherein the detection means comprises a cover member, and wherein the chamber means has an internal wall surface with a surface roughness that is larger than a surface roughness of a surface of the cover member that faces the chamber means.

10. An apparatus as claimed in any preceding claim, wherein the detection means comprises a cover member with a surface of the cover member facing the chamber means having a water repellent treatment.

11. An apparatus as claimed in any preceding claim, wherein the detection means comprises a first sensor in a first cover and a second sensor in a second cover, with the first cover having a gas introducing hole and the second sensor being a reference sensor with the second cover containing a reference gas.

12. An apparatus as claimed in claim 11, wherein the first and second cover members:
are spaced apart with a gap therebetween; and/or
are spaced inwardly from an internal side wall of the chamber means.

13. A fuel cell system comprising:
a fuel cell with a fuel electrode;
a pipe arranged to supply a gas to the fuel electrode of the fuel cell;
a pipe arranged to discharge the gas from the fuel electrode; and
an apparatus as claimed in any preceding claim connected to at least one of the pipes.

14. A fuel cell system comprising a fuel cell that receives hydrogen and an apparatus as claimed in any of claims 1 to 12 arranged in an area to detect leakage of hydrogen from the fuel cell system.

15. A vehicle, comprising:
a vehicle body supporting a fuel cell system as claimed in claim 13 or claim 14; and
at least one drive wheel arranged to use electric power generated by the fuel cell system as a drive source.
